# FASCICULE DE BREVET EUROPEEN

(11) **EP 1 163 909 B1**
(45) Date de publication et mention de la délivrance du brevet: **15.10.2008**
(21) Numéro de dépôt: 01401415.3
(22) Date de dépôt: 31.05.2001
(51) Int. Cl.: A61K 39/39

(54) **Utilisation d'éthers de sucres, comme adjuvants d'immunité dans les compositions vaccinales, compositions thérapeutiques les renfermant et leur utilisation comme vaccins**
Zuckerether als Immunoadjuvantien in Impfstoffzusammensetzungen, diese enthaltende therapeutische Zusammensetzungen
Use of sugar ethers as immunity adjuvant in vaccine compositions, therapeutic compositions containing them and their use as vaccine

(30) Priorité: 09.06.2000 FR 0007408
(43) Date de publication de la demande: 19.12.2001
(73) Titulaire: SOCIETE D'EXPLOITATION DE PRODUITS POUR LES INDUSTRIES CHIMIQUES-SEPPIC, F-75321 Paris Cédex 07 (FR)
(72) Inventeur: Aucouturier, Jérôme, 92290 Chatenay Malabry (FR); Dupuis, Laurent, 81100 Castres (FR); Ganne, Vincent, 94210 La Varenne (Saint Hilaire) (FR); Deville, Sébastien, 75011 Paris (FR); Trouve, Gérard, 81100 Castres (FR)
(74) Mandataire: Conan, Philippe Claude

(56) Documents cités:
- WO-A-91/00107
- WO-A-94/16681
- WO-A-95/11700
- WO-A-96/32964
- FR-A- 2 754 182

## Description

La présente invention concerne de nouveaux adjuvants pour compositions vaccinales ainsi que des compositions comprenant au moins un antigène, notamment un antigène d'origine virale, bactérienne ou parasitaire et au moins un adjuvant.

Le développement de vaccins inactivés ou contenant des antigènes purifiés est de plus en plus important, car il permet d'éviter les effets secondaires indésirables. Cependant, l'amélioration de la qualité des antigènes se fait au détriment de leur caractère immunogène. C'est pour cette raison qu'ils sont associés à des adjuvants d'immunité.

Les adjuvants d'immunité sont des produits qui augmentent les réactions du système immunitaire, lorsqu'ils sont administrés en présence d'antigènes d'origine virale, bactérienne ou parasitaire. Ils provoquent l'apparition massive de macrophages au site d'injection, puis dans les nodules lymphatiques, accroissent la production d'immunoglobulines spécifiques, les anticorps, et stimulent de nombreuses cellules impliquées dans les mécanismes de la défense immunitaire.

Ces adjuvants sont de natures diverses. Ils peuvent par exemple consister en des mélanges d'huiles et de tensioactifs conduisant à des vaccins sous forme de liposomes ou d'émulsions.

Les adjuvants de Freund sont très efficaces ; ils résultent de l'association d'une huile minérale et d'un ester de mannitol contenant ou non une mycobactérie tuée.

Les vaccins réalisés par mélange à parts égales, entre un adjuvant de Freund et un milieu antigénique aqueux, restent encore les références dans le monde entier pour les études en laboratoire. Ils se présentent sous forme d'émulsions eau dans huile (E/H), c'est à dire d'émulsions dans lesquelles la phase continue est l'huile. Or, ces émulsions sont très visqueuses et sont donc difficilement injectables. Elles sont aussi peu stables, puisque des déphasages sont observés quelques jours seulement, après leur préparation.

Comme adjuvants usuels, il y a aussi des sels de métaux, tels que l'hydroxyde d'aluminium, le nitrate de cérium, le sulfate de zinc, l'hydroxyde de fer colloïdal ou le chlorure de calcium. Parmi ceux-ci, l'hydroxyde d'aluminium est le plus couramment utilisé. Ces adjuvants sont décrits dans l'article de Rajesh K. Gupta et al "Adjuvants, balance between toxicity and adjuvanticity", Vaccine, vol. 11, Issue 3, 1993, pages 293-306. Ils présentent cependant une faible efficacité immunostimulante et induisent parfois, lorsque ces compositions thérapeutiques sont injectées, la formation de lésions et autres réactions locales, telles que les granulomes, au point d'injection.

On a découvert plus récemment, que les sels hydrosolubles de métaux divalents ou trivalents, étaient de bons adjuvants de l'immunité. Parmi ceux-ci, le gluconate de manganèse, le gluconate de calcium, le glycérophosphate de manganèse, l'acétate d'aluminium soluble ou le salicylate d'aluminium sont les plus prometteurs. De tels adjuvants sont décrits dans les demandes internationales de brevet publiées sous les numéros WO 96/32964 et WO 98/17311.

Comme autres adjuvants de l'immunité, notamment dans le cas de l'administration mucosale, on peut citer les composés sympathomimétiques décrits dans la demande internationale de brevet publiée sous le numéro WO 98/15288.

A l'occasion de recherches sur la mise au point de nouveaux adjuvants, la demanderesse a découvert que certains agents tensioactifs eux-mêmes, possédaient une efficacité immunostimulante en l'absence d'huiles, et que l'on pouvait ainsi préparer des compositions vaccinales aqueuses, comprenant un ou plusieurs de ces agents comme immunostimulants.

C'est pourquoi, l'invention a pour objet, l'utilisation d'un composé de formule (I) :

R₁-O-(G)ₓ-H (I)

dans laquelle R₁ représente un radical hydrocarboné linéaire ou ramifié, saturé ou insaturé comprenant de 1 à 30 atomes de carbones, G représente le reste d'un saccharide et x représente un nombre décimal compris entre 1 et 5 ou un mélange de composés de formule (I), comme adjuvant d'immunité dans une composition vaccinale comprenant au moins un antigène ou au moins un générateur in vivo d'un composé comprenant une séquence d'acides aminés.

Par reste d'un saccharide, on désigne pour G, un radical bivalent résultant de l'enlèvement sur une molécule de sucre, d'une part d'un atome d'hydrogène d'un de ses groupes hydroxyle et d'autre part du groupe hydroxyle anomérique. Le terme saccharide désigne notamment le glucose ou dextrose, le fructose, le mannose, le galactose, l'altrose, l'idose, l'arabinose, le xylose, le ribose, le gulose, le lyxose, le maltose, le maltotriose, le lactose, le cellobiose, le dextrane, le talose, l'allose, le raffinose, le lévoglucane, la cellulose ou l'amidon. La structure oligomérique (G)ₓ peut se présenter sous toute forme d'isomérie, qu'il s'agisse d'isomérie optique, d'isomérie géométrique ou d'isomérie de position, elle peut aussi représenter un mélange d'isomères.

Le nombre x, qui représente dans la formule (I) le degré moyen de polymérisation du saccharide, est plus particulièrement compris entre 1 et 3, notamment entre 1,05 et 2,5, tout particulièrement entre 1,1 et 2,0 et de préférence, inférieur ou égal à 1,5.

G représente plus particulièrement le reste du glucose ou le reste du xylose.

Le radical R₁ représente notamment un radical comportant de 5 à 22 atomes de carbone choisi parmi les radicaux pentyle, hexyle, heptyle, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle, lesdits radicaux étant linéaires ou ramifiés. R₁ représente de préférence un radical comportant de 8 à 20 atomes de carbone lesdits radicaux étant linéaires ou ramifiés.

R₂ représente plus particulièrement un radical comportant de 8 à 22 atomes de carbone choisi parmi les radicaux, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle, lesdits radicaux étant linéaires ou ramifiés.

Par antigène ou au moins un générateur in vivo d'un composé comprenant une séquence d'acides aminés, on désigne soit des microorganismes tués, tels que les virus, les bactéries ou les parasites, soit des fractions purifiées de ces micro-organismes, soit des micro-organismes vivants dont le pouvoir pathogène a été atténué. A titre de virus pouvant constituer un antigène selon la présente invention, on peut citer le virus de la rage, les herpès virus, tels que le virus de la maladie d'Aujeszky, les orthomixovirus tels que Influenzae, les picornavirus tels que le virus de la fièvre aphteuse ou les rétrovirus tels que les VIH. A titre de micro-organisme du type bactérien pouvant constituer un antigène selon la présente invention, on peut citer E. Coli, et ceux des genres Pasteurella, Furonculosis, Vibriosis, Staphylococcus et Streptococcus. A titre de parasite, on peut citer ceux des genres Trypanosoma, Plasmodium et Leishmania. On peut aussi citer les virus recombinants notamment les virus non enveloppés tels que les adénovirus, le virus de la vaccine, le virus Canarypox, les herpès virus ou les baculovirus. On désigne aussi un vecteur recombinant viral non enveloppé vivant, dont le génome contient, insérée de préférence dans une partie non essentielle pour la réplication du virus enveloppé correspondant, une séquence codant pour une sous-unité antigénique induisant une synthèse d'anticorps et/ou un effet protecteur contre le susdit virus enveloppé ou micro-organisme pathogène ; ces sous-unités antigéniques peuvent être par exemple, une protéine, une glycoprotéine, un peptide ou une fraction peptidique et/ou protectrice contre une infection par un micro-organisme vivant tel un virus enveloppé, une bactérie ou un parasite. Le gène exogène inséré dans le micro-organisme peut être, par exemple, issu d'un virus Aujeszky ou HIV.

On peut citer notamment un plasmide recombinant constitué d'une séquence de nucléotides, dans laquelle est insérée une séquence nucléotidique exogène, provenant d'un micro-organisme ou d'un virus pathogène. Cette dernière séquence nucléotidique a pour but de permettre l'expression d'un composé comprenant une séquence d'acides aminés, ce composé ayant lui-même pour but de déclencher une réaction immune dans un organisme hôte. Par générateur "in vivo" d'un composé comprenant une séquence d'acides aminés, on désigne tout produit biologique capable d'exprimer ledit composé dans l'organisme hôte dans lequel on a introduit ledit générateur in vivo. Le composé comprenant la séquence d'acides aminés, peut être une protéine, un peptide ou une glycoprotéine. Ces générateurs in vivo sont généralement obtenus par des procédés issus du génie génétique. Plus particulièrement, ils peuvent consister en des micro-organismes vivants, généralement un virus, jouant le rôle de vecteur recombinant, dans lequel est insérée une séquence nucléotidique, notamment un gène exogène. Ces composés sont connus en tant que tels et utilisés notamment comme vaccin sous unitaire recombinant. A cet égard, on peut se référer à l'Article de M. ELOIT et al., Journal of Virology (1990) 71, 2925-2431 et aux demandes internationales de brevet publiées sous les numéros WO-A-91/00107 et WO-A-94/16681. Les générateurs in vivo selon l'invention peuvent aussi consister en un plasmide recombinant comprenant une séquence nucléotidique exogène, capable d'exprimer dans un organisme hôte un composé comprenant une séquence d'acides aminés. De tels plasmides recombinants et leur mode d'administration dans un organisme hôte ont été décrits en 1990, par LIN et al. , Circulation 82:2217,2221 ; COX et al., J. of VIROL, Sept. 1993, 67, 9, 5664-5667 et dans la demande internationale publiée sous le numéro WO 95/25542. Selon la nature de la séquence nucléotidique comprise dans le générateur in vivo, le composé comprenant la séquence d'acides aminés qui est exprimé au sein de l'organisme hôte, peut :
(i) être un antigène, et permettre le déclenchement d'une réaction immune, leukine 2. Celles-ci permettent le déclenchement ou le renforcement d'une réaction immune visant à l'élimination sélective des cellules cancéreuses.

Selon un premier aspect particulier de la présente invention, celle-ci a pour objet, l'utilisation telle que définie précédemment, dans une composition vaccinale comprenant une phase aqueuse et une ou plusieurs huiles choisies parmi les huiles minérales, animales ou végétales, dans une proportion pondérale en phase huileuse, comprise en 10% et 90% de son poids total et de préférence entre 30% et 70% en poids de son poids total

Lorsque la composition vaccinale comprend une phase aqueuse et une phase huileuse, elle est généralement sous forme d'une émulsion eau dans huile (E/H), d'une émulsion huile dans eau (H/E), d'une émulsion eau dans huile dans eau (E/H/E) ou d'une microémulsion.

Selon un deuxième aspect particulier de la présente invention, celle-ci a pour objet, l'utilisation d'un mélange comprenant :
a) un composé de formule (I):

   R₁-O-(G)ₓ-H (I)

   telle que définie précédemment, ou un mélange de composés de formule (I), et si désiré
b) un composé de formule (II) :

   R₂-OH (II)
dans laquelle R₂ représente indépendamment de R₁, un radical hydrocarboné linéaire ou ramifié, saturé ou insaturé comprenant de 8 à 30 atomes de carbonesou un mélange de composés de formule (II), comme adjuvant d'immunité dans une composition vaccinale comprenant une phase aqueuse et ne comprenant pas de phase huileuse.

L'invention a aussi pour objet une composition sous forme d'une phase aqueuse comprenant :
(i) au moins un antigène ou au moins un générateur in vivo d'un composé comprenant une séquence d'acides aminés et,
(ii) au moins un composé de formule (I) :

   R₁-O-(G)ₓ-H (I)

   et si désiré
(iii) au moins un composé de formule (II),
   pour laquelle les formules (I) et (II) ont les significations indiquées précédemment.

(i) au moins un antigène ou au moins un générateur in vivo d'un composé comprenant une séquence d'acides aminés et,
(ii) au moins un composé de formule (I)

   R₁-O-(G)ₓ-H (I)

   et
(iii) au moins un composé de formule (II),
pour laquelle les formules (I) et (II) ont les significations indiquées précédemment.

Lorsque la composition telle que définie précédemment comporte au moins un composé de formule (I) et au moins un composé de formule (II), le rapport pondéral composé de formule (I) / composé de formule (II) est généralement compris entre 10 / 90 et 90 / 10, plus particulièrement entre 10 / 90 et 60 / 40.

La composition telle que définie précédemment, peut comprendre en outre une ou plusieurs huiles choisies parmi les huiles minérales, animales ou végétales, dans une proportion pondérale en phase huileuse, comprise en 10 et 90% de son poids total et de préférence entre 30% et 70% en poids de son poids total.

Lorsque la composition comprend une phase aqueuse et une phase huileuse, elle est généralement sous forme d'une émulsion eau-dans huile (E/H), d'une émulsion huile dans eau (H/E), d'une émulsion eau dans huile dans eau (E/H/E) ou d'une microémulsion.

Une composition telle que définie précédemment, peut aussi comprendre une phase aqueuse et ne pas comprendre de phase huileuse.

Une composition selon l'invention comprend une concentration en antigène qui dépend de la nature de cet antigène et de la nature du sujet traité. Il est toutefois particulièrement remarquable qu'un adjuvant selon l'invention, permette de diminuer notablement la dose habituelle d'antigène requise. La concentration adéquate d'antigène peut être déterminée de manière classique par l'homme du métier. Généralement, cette dose est de l'ordre de 0,1 µg / cm³ à 1 g / cm³ plus généralement comprise entre 1 µg / cm³ et 100 mg / cm³.

La concentration dudit générateur in vivo dans la composition selon l'invention dépend, là encore, notamment de la nature dudit générateur et de l'hôte dans lequel il est administré. Cette concentration peut être aisément déterminée par l'homme du métier, sur la base d'expérience de routine. A titre indicatif, on peut toutefois préciser que lorsque le générateur in vivo est un microorganisme recombinant, sa concentration dans la composition selon l'invention peut être comprise entre 10² et 10¹⁵ micro-organismes / cm³, de préférence entre 10⁵ et 10¹² micro-organismes / cm³. Lorsque le générateur in vivo est un plasmide recombinant, sa concentration dans la composition est comprise entre 0,01 mg et 100 mg / cm³.

Une composition objet de la présente invention, contient entre 0,2 mg / cm³ et 500 mg / cm³ d'adjuvant, plus particulièrement entre 2 mg / cm³ et 500 mg / cm³ d'adjuvant et de préférence entre 50 mg / cm³ et 200 mg / cm³ d'adjuvant.

Les composés de formules (I) et (II) sont de préférence pharmaceutiquement acceptables et bien tolérés par l'organisme humain ou animal ; ils doivent notamment être dépourvus de métaux lourds et présenter des indices d'acides ou de peroxydes très faibles. Il est également souhaitable qu'ils satisfassent aux normes déterminées par des tests d'innocuité, notamment ceux décrits par S.S. Berllin, Annales of Allergy, 1962, 20, 473 ou ceux décrits dans la Pharmacopée européenne.

La composition selon l'invention, peut aussi comporter un agent stimulant immunitaire conventionnel tel l'Avridine^{™}, la N,N-dioctadecyl-N',N'-bis(2-hydroxyéthyl) propanediamine, les dérivés du MDP (muramyl dipeptide), notamment le thréonyl-MDP, les dérivés de l'acide mycolique ou les dérivés du Lipide A.

La composition selon l'invention, peut aussi comprendre un ou plusieurs sels organiques de cations métalliques hydrosolubles, tel que par exemple le gluconate de calcium, le gluconate de manganèse, le glycérophosphate de manganèse, le glycérophosphate de calcium, le fructoheptonate de calcium, le fructoheptonate de manganèse, le salicylate d'aluminium ou l'acétate d'aluminium soluble.

Lorsque la composition adjuvante selon l'invention comprend un ou plusieurs sels, celui-ci est à une concentration de 0,02 à 3000 mg / cm³, de préférence 0,1 mg à 1000 mg / cm³, plus préférentiellement de 0,1 mg à 150 mg / cm³. La composition selon l'invention, peut comprendre un composé sympathomimétique.

Par composés sympathomimétiques, on désigne notamment les amphétamines, les catécholamines, les phénylisopropylamines ou la tyramine. Comme exemples de tels composés, on peut citer notamment l'isoprotérénol, la L-Epinephrine, le lévartérénol, l'éphédrine, la phényléphédrine ou le salbutamol. Lorsque la composition adjuvante selon l'invention comprend un composé sympathomimétique, celui-ci est à une concentration de 10⁻¹⁰ molaire à 10⁻² molaire, de préférence de 10⁻⁷ molaire à 10⁻⁵ molaire.

La composition selon l'invention peut être utilisée comme médicament préventif ou curatif. Selon la nature de l'antigène ou du générateur in vivo, une composition selon l'invention peut être administrée à des poissons, des crustacés tels que les crevettes, des volailles, notamment, des oies, des dindes, des pigeons et des poulets, aux canidés tels le chien, aux félidés tels le chat, aux porcs, aux primates, aux bovidés, aux ovidés et aux chevaux. La composition selon l'invention peut être également administrée à l'homme. L'administration de la composition peut se faire de manière classique par voie parentérale, notamment par injection sous-cutanée, intramusculaire ou intrapéritonéale ou par voie mucosale notamment par voie orale, voie rectale, voie nasale, voie vaginale. Selon un autre aspect de l'invention, celle-ci consiste en l'utilisation d'un adjuvant tel que défini ci-dessus pour la préparation d'un vaccin destiné à la prévention ou au traitement d'une maladie infectieuse, notamment une maladie infectieuse engendrée par un virus ou un micro-organisme tels ceux mentionnés plus haut.

Selon un autre dernier aspect de la présente invention, celle-ci consiste en l'utilisation de cet adjuvant pour la préparation d'une composition destinée à soigner une maladie d'ordre fonctionnel, telle le cancer ou la mucoviscidose.

Les exemples suivants illustrent l'invention sans toutefois la limiter.

### A) tensioactifs utilisés

Les tensioactifs utilisés sont les suivants :

| composition % pondéraux | Alkyl polyglucosides de formule (I) | Alcools de formule (II) | Eau |
|---|---|---|---|
| composition (A) | ~12% R₁ en C₁₆ et C₁₈ | ~87% R₂ en C₁₆ et C₁₈ | 0% |
| composition (B) | ~14,5% R₁ en C₁₂ + ~19% R₁ en C₁₄ + ~11 % R₁ en C₁₆ + ~21,5% R₁ en C₁₈ | ~1,5% R₂ en C₁₄ + ~4,5% R₂ en C₁₆ + ~28% R₂ en C₁₈ | 0% |
| Composition C | 50-55% R₁ en C₁₀ - C₁₄ | < 5% | 40-45% |
| Composition D | ~14,5% R₁ isostéaryle | ~85,5% R₂ isostéaryle | 0% |
| Composition E | ~11% R₁ isostéaryle | ~90% R₂ isostéaryle | 0% |
| Composition F | ~16,5% R₁ oléyle | ~83,5% R₂ oléyle | 0% |

### Exemple 1

On a injecté par voie sous-cutanée, à différents lots de 5 souris femelles de souche OF1 ayant un poids moyen de 18 à 20 grammes, 100 microlitres de différentes compositions contenant un agent tensioactif une huile, du tampon phosphate (PBS) et 0,1 mg / cm³ d'ovalbumine, à t = 0 avec un rappel à t = 28 jours. On effectue les prélèvements sanguins à 14, 28, 42, 56 et 90 jours. On procède à des dosages ELISA sur les prélèvements sanguins, des IgG1 pour déterminer la réponse immunitaire humorale et les IgG2a, pour déterminer la réponse immunitaire cellulaire.

Les compositions injectées sont les suivantes :

| compositions injectées | phase grasse | PBS | Ovalbumine 0,1 mg / cm³ |
|---|---|---|---|
| composition 1 | 1 ml contenant 10% de composition D + 90% de MARCOL ^{™} 52 | 1 ml | 20 µl |
| composition 2 | 1 ml contenant 10% de composition E + 90% de MARCOL ^{™} 52 | 1 ml | 20 µl |
| composition 3 | 1 ml contenant 10% de composition F + 90% de MARCOL ^{™} 52 | 1 ml | 20 µl |
| témoin | 1 ml de MARCOL^{™} 52 | 1 ml | 20 µl |

Les résultats des tests ELISA sont les suivants :

| | **Dosage des IgG1 (échelle de temps en jours)** | | | | |
|---|---|---|---|---|---|
| composition injectée | **J14** | **J28** | **J42** | **J56** | **J90** |
| composition 1 | 30000 | 300000 | 1000000 | 700000 | 700000 |
| composition 2 | 30000 | 300000 | 1000000 | 1000000 | 700000 |
| Composition 3 | 15000 | 100000 | 900000 | 600000 | 700000 |
| témoin | 0 | 0 | 80000 | 15000 | 15000 |

| | **Dosage des IgG2 (échelle de temps en jours)** | | | | |
|---|---|---|---|---|---|
| composition injectée | **J14** | **J28** | **J42** | **J56** | **J90** |
| composition 1 | <1000 | 2500 | 100000 | 30000 | 9000 |
| composition 2 | <1000 | 6000 | 100000 | 50000 | 30000 |
| Composition 3 | <1000 | 6000 | 100000 | 50000 | 30000 |
| témoin | <1000 | <1000 | <1000 | <1000 | <1000 |

### Exemple 2

On a injecté par voie sous-cutanée, à différents lots de 5 souris femelles de souche OF1 ayant un poids moyen de 18 à 20 grammes, 100 microlitres de différentes compositions contenant un agent tensioactif, du tampon phosphate (PBS) et 25 mg / cm³ d'albumine BSA, à t = 0 avec un rappel à t = 28 jours. On effectue les prélèvements sanguins à 14, 28, 42, 56, 90 et 180.jours

On procède à des dosages ELISA sur les prélèvements sanguins, des IgG1 pour déterminer la réponse immunitaire humorale et les IgG2a, pour déterminer la réponse immunitaire cellulaire. Les compositions injectées sont les suivantes :

| compositions injectées | Adjuvant | PBS | BSA |
|---|---|---|---|
| composition 4 | 0,2 ml d'une solution dans le PBS, comprenant 10% en poids de la composition A. | 1,8 ml | 0,02 ml |
| composition 5 | 0,02 ml d'une solution dans le PBS, comprenant 14,3% en poids de la composition B. | 1,98 ml | 0,02 ml |
| composition 6 | 0,02 ml d'une solution dans le PBS, comprenant 20% en poids de la composition C. | 1,98 ml | 0;02 ml |
| composition 7 | 2 ml d'une solution dans le PBS, comprenant 10% en poids de la composition A. | 1,8 ml | 0,02 ml |
| composition 8 | 0,2 ml d'une solution dans le PBS, comprenant 14,3% en poids de la composition B. | 1,8 ml | 0,02 ml |
| composition 9 | 0,2 ml d'une solution dans le PBS, comprenant 20% en poids de la composition C. | 1,8 ml | 0,02 ml |
| Témoin | 0 | 2ml | 0,02 ml |

Les résultats des tests ELISA sont les suivants :

| | **Dosage des IgG1 (échelle de temps en jours)** | | | | | |
|---|---|---|---|---|---|---|
| composition injectée | **J14** | **J28** | **J42** | **J56** | **J90** | **J180** |
| Composition 4 | 1500 | 6000 | 128000 | 64000 | 8000 | 12000 |
| Composition 7 | 8000 | 12000 | 128000 | 96000 | 12000 | 4000 |
| Composition 5 | 1000 | 1000 | 32000 | 24000 | 3000 | 3000 |
| Composition 8 | <1000 | 1500 | 64000 | 32000 | 12000 | 16000 |
| Composition 6 | <1000 | <1000 | 12000 | 12000 | 4000 | <1000 |
| Composition 9 | <1000 | 1500 | 24000 | 12000 | 3000 | <1000 |
| témoin | <1000 | <1000 | 4000 | 6000 | <1000 | <1000 |

| | **Dosage des IgG2 (échelle de temps en jours)** | | | | | |
|---|---|---|---|---|---|---|
| composition injectée | **J14** | **J28** | **J42** | **J56** | **J90** | **J180** |
| Composition 4 | <1000 | 2000 | 16000 | 8000 | 12000 | 8000 |
| Composition 7 | <1000 | <1000 | 4000 | 3000 | 4000 | <1000 |
| Composition 5 | <1000 | <1000 | <1000 | <1000 | <1000 | <1000 |
| Composition 8 | <1000 | <1000 | <1000 | <1000 | <1000 | 16000 |
| Composition 6 | <1000 | <1000 | 3000 | 2000 | 2000 | <1000 |
| Composition 9 | <1000 | <1000 | 6000 | 3000 | 4000 | 2000 |
| témoin | <1000 | <1000 | <1000 | <1000 | <1000 | <1000 |

Les résultats exposés ci-dessous mettent en évidence l'activité immunostimulantes des composés de formule(I) seuls ou en association avec les composés de formule (II).

## Revendications

1. Utilisation d'un composé de formule (I) :
R₁-O-(G)ₓ-H (I)
dans laquelle R₁ représente un radical hydrocarboné linéaire ou ramifié, saturé ou insaturé comprenant de 1 à 30 atomes de carbones, G représente le reste d'un saccharide et x représente un nombre décimal compris entre 1 et 5 ou un mélange de composés de formule (I), comme adjuvant d'immunité dans une composition vaccinale comprenant au moins un antigène ou au moins un générateur in vivo d'un composé comprenant une séquence d'acides aminés.

2. Utilisation telle que définie à la revendication 1, selon laquelle dans la formule (I), le nombre x est compris entre 1 et 3, plus particulièrement entre 1,05 et 2,5, tout particulièrement entre 1,1 et 2,0 et de préférence, inférieur ou égal à 1,5.

3. Utilisation telle que définie à l'une des revendications 1 ou 2, selon laquelle dans la formule (I), G représente le reste du glucose ou le reste du xylose.

4. Utilisation telle que définie à l'une quelconque des revendications 1 à 3, selon laquelle dans la formule (I), le radical R₁ représente un radical comportant de 5 à 22 atomes de carbone choisi parmi les radicaux pentyle, hexyle, heptyle, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle, lesdits radicaux étant linéaires ou ramifiés.

5. Utilisation telle que définie à la revendication 4, selon laquelle dans la formule (1), R₁ représente un radical comportant de 8 à 20 atomes de carbone lesdits radicaux étant linéaires ou ramifiés.

6. Utilisation telle que définie à l'une quelconque des revendications 1 à 5, selon laquelle dans la formule (I), R₂ représente un radical comportant de 8 à 22 atomes de carbone choisi parmi les radicaux, octyle, nonyle, décyle, undécyle, dodécyle, tridécyle, tétradécyle, pentadécyle, hexadécyle, heptadécyle, octadécyle, nonadécyle, eicosyle, uneicosyle, docosyle, heptadécènyle, eicosènyle, uneicosènyle, docosènyle ou heptadécadiènyle ou décènyle, lesdits radicaux étant linéaires ou ramifiés.

7. Utilisation telle que définie à l'une quelconque des revendications 1 à 6, dans une composition vaccinale comprenant une phase aqueuse et une ou plusieurs huiles choisies parmi les huiles minérales, animales ou végétales dans une proportion pondérale en phase huileuse, comprise en 10% et 90% de son poids total et de préférence entre 30% et 70% en poids de son poids total.

8. Utilisation telle que définie à l'une quelconque des revendications 1 à 7, dans une composition vaccinale comprenant une phase aqueuse et ne comprenant pas de phase huileuse.

9. Composition comprenant :
(i) au moins un antigène ou au moins un générateur in vivo d'un composé comprenant une séquence d'acides aminés et,
(ii) au moins un composé de formule (I) :
R₁-O-(G)ₓ-H (I)
et
(iii) au moins un composé de formule (II),
R₂-OH (II)
dans laquelle R₂ représente indépendamment de R₁, un radical hydrocarboné linéaire ou ramifié, saturé ou insaturé comprenant de 8 à 30 atomes de carbones, dans laquelle la formules (I) est telle que définie à l'une quelconques des revendications 1 à 6.

10. Composition telle que définie à la revendication 9, comprenant en outre une ou plusieurs huiles choisies parmi les huiles minérales, animales ou végétale, dans une proportion pondérale en phase huileuse, comprise en 10% et 90% de son poids total et de préférence entre 30% et 70% en poids de son poids total.

11. Composition telle que définie à la revendication 10, sous forme d'une émulsion eau-dans huile (E/H), d'une émulsion huile dans eau (H/E), d'une émulsion eau dans huile dans eau (E/H/E) ou d'une microémulsion.

12. Composition telle que définie à la revendication 9, ne pas comprenant de phase huileuse.

13. Composition telle que définie à l'une des revendications 9 à 12, comprenant en outre un ou plusieurs sels organiques de cations métalliques hydrosolubles choisis parmi le gluconate de calcium, le gluconate de manganèse, le glycérophosphate de manganèse, le glycérophosphate de calcium, le fructoheptonate de calcium, le fructoheptonate de manganèse, le salicylate d'aluminium ou l'acétate d'aluminium soluble.

14. Composition telle que définie à l'une des revendications 9 à 13, comprenant en outre un composé sympathomimétique. choisi parmi la tyramine. l'isoprotérénol, la L-Epinéphrine, le lévartérénol, l'éphédrine, la phényléphédrine ou le salbutamol.

15. Composition telle que définie à l'une des revendications 9 à 14, pour la mise en oeuvre d'un traitement thérapeutique du corps humain ou animal.

16. Composition telle que définie à la revendication 15, pour la mise en oeuvre d'un traitement thérapeutique du corps humain ou animal par voie parentérale, par injection sous-cutanée, intramusculaire ou intrapéritonéale

17. Composition telle que définie à la revendication 16, pour la mise en oeuvre d'un traitement thérapeutique du corps humain ou animal par voie mucosale notamment par voie orale, voie rectale, voie nasale, voie vaginale.

## Claims

1. Use of a compound of formula (I):
R₁-O-(G)ₓ-H (I)
in which R₁ represents a saturated or unsaturated, linear or branched hydrocarbon-based radical comprising 1 to 30 carbon atoms, G represents the residue of a saccharide and x represents a decimal number between 1 and 5, or a mixture of compounds of formula (I), as an adjuvant of immunity in an immunization composition comprising at least one antigen or at least one in vivo generator of a compound comprising an amino acid sequence.

2. Use as defined in Claim 1, according to which, in formula (I), the number x is between 1 and 3, more particularly between 1.05 and 2.5, most particularly between 1.1 and 2.0, and preferably less than or equal to 1.5.

3. Use as defined in either of Claims 1 and 2, according to which, in formula (I), G represents the residue of glucose or the residue of xylose.

4. Use as defined in any one of Claims 1 to 3, according to which in formula (I), the R₁ radical represents a radical comprising from 5 to 22 carbon atoms, chosen from pentyl, hexyl, heptyl, octyl, nonyl, decyl, undecyl, dodecyl, tridecyl, tetradecyl, pentadecyl, hexadecyl, heptadecyl, octadecyl, nonadecyl, eicosyl, uneicosyl, docosyl, heptadecenyl, eicosenyl, uneicosenyl, docosenyl or heptadecadienyl or decenyl radicals, said radicals being linear or branched.

5. Use as defined in Claim 4, according to which, in formula (I), R₁ represents a radical comprising from 8 to 20 carbon atoms, said radicals being linear or branched.

6. Use as defined in any one of Claims 1 to 5, according to which, in formula (I), R₂ represents a radical comprising from 8 to 22 carbon atoms, chosen from octyl, nonyl, decyl, undecyl, dodecyl, tridecyl, tetradecyl, pentadecyl, hexadecyl, heptadecyl, octadecyl, nonadecyl, eicosyl, uneicosyl, docosyl, heptadecenyl, eicosenyl, uneicosenyl, docosenyl or heptadecadienyl or decenyl radicals, said radicals being linear or branched.

7. Use as defined in any one of Claims 1 to 6, an immunization composition comprising an aqueous phase and one or more oils chosen from mineral, animal or plant oils, in a weight proportion in oily phase of between 10 and 90% of the total weight thereof, and preferably between 30% and 70% by weight of the total weight thereof.

8. Use as defined in any one of Claims 1 to 7, in an immunization composition comprising an aqueous phase and not comprising an oily phase.

9. Composition comprising:
(i) at least one antigen or at least one in vivo generator of a compound comprising an amino acid sequence and,
(ii) at least one compound of formula (I):
R₁-O- (G)ₓ-H (I)
and,
(iii) at least one compound of formula (II),
R₂-OH (II)
in which R₂ represents, independently of R₁, a saturated or unsaturated, linear or branched hydrocarbon-based radical comprising from 8 to 30 carbon atoms,
in which formula (I) is as defined in any one of Claims 1 to 6.

10. Composition as defined in Claim 9, also comprising one or more oils chosen from mineral, animal or plant oils, in a weight proportion in oily phase of between 10 and 90% of the total weight thereof, and preferably between 30% and 70% by weight of the total weight thereof.

11. Composition as defined in Claim 10, in the form of a water-in-oil (W/O) emulsion, of an oil-in-water (O/W) emulsion, of a water-in-oil-in-water (W/O/W) emulsion or of a microemulsion.

12. Composition as defined in Claim 9, not comprising an oily phase.

13. Composition as defined in one of Claims 9 to 12, also comprising one or more water-soluble metal cation organic salts, such as for example calcium gluconate, manganese gluconate, manganese glycerophosphate, calcium glycerophosphate, calcium fructoheptonate, manganese fructoheptanate, aluminium salicylate or soluble aluminium acetate.

14. Composition as defined in one of Claims 9 to 13, also comprising a sympathomimetic compound chosen from tyramine, isoproterenol, L-adrenaline, levarterenol, ephedrine, phenylephedrine or salbutamol.

15. Composition as defined in one of Claims 9 to 14, for carrying out therapeutic treatment of the human or animal body.

16. Composition as defined in Claim 15, for carrying out therapeutic treatment of the human or animal body via the parenteral pathway, by subcutaneous, intramuscular or intraperitoneal injection.

17. Composition as defined in Claim 16, for carrying out therapeutic treatment of the human or animal body via the mucosal pathway, in particular orally, rectally, nasally or vaginally.

## Patentansprüche

1. Verwendung einer Verbindung nach Formel (I):
R₁ -O- (G)ₓ- H (I)
wobei R₁ für einen geradkettigen oder verzweigten, gesättigten oder ungesättigten Kohlenwasserstoffrest umfassend 1 bis 30 Kohlenstoffatome steht, G für den Rest eines Saccharids steht und x für eine Dezimalzahl von 1 bis 5 steht, oder einer Mischung von Verbindungen nach Formel (I) als Immunoadjuvantien in einer Impfstoffzusammensetzung, die mindestens ein Antigen umfasst oder die mindestens ein Mittel umfasst, welches in vivo eine Verbindung erzeugt, die eine Aminosäuresequenz umfasst.

2. Verwendung gemäß der Begriffsbestimmung in Anspruch 1, wobei in der Formel (I) die Zahl x im Bereich von 1 bis 3 liegt, insbesondere im Bereich von 1,05 bis 2,5 und ganz besonders von 1,1 bis 2,0, wobei sie vorzugsweise kleiner oder gleich 1,5 ist.

3. Verwendung gemäß der Begriffsbestimmung in einem der Ansprüche 1 bis 2, wobei in der Formel (I) das Symbol G für den Glucoserest oder den Xyloserest steht.

4. Verwendung gemäß der Begriffsbestimmung in einem beliebigen der Ansprüche 1 bis 3, wobei in der Formel (I) der Rest R₁ für einen Rest mit 5 bis 22 Kohlenstoffatomen steht, welcher aus den Resten Pentyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Uneicosyl, Docosyl, Heptadecenyl, Eicosenyl, Uneicosenyl, Docosenyl oder Heptadecadienyl oder Decenyl gewählt ist, wobei die Reste geradkettig oder verzweigt sind.

5. Verwendung gemäß der Begriffsbestimmung in Anspruch 4, wobei in der Formel (I) das Symbol R₁ für einen Rest mit 8 bis 20 Kohlenstoffatomen steht, wobei die Reste geradkettig oder verzweigt sind.

6. Verwendung gemäß der Begriffsbestimmung in einem beliebigen der Ansprüche 1 bis 5, wobei in der Formel (I) der Rest R₁ für einen Rest mit 8 bis 22 Kohlenstoffatomen steht, welcher aus den Resten Octyl, Nonyl, Decyl, Undecyl, Dodecyl, Tridecyl, Tetradecyl, Pentadecyl, Hexadecyl, Heptadecyl, Octadecyl, Nonadecyl, Eicosyl, Uneicosyl, Docosyl, Heptadecenyl, Eicosenyl, Uneicosenyl, Docosenyl oder Heptadecadienyl oder Decenyl gewählt ist, wobei die Reste geradkettig oder verzweigt sind.

7. Verwendung gemäß der Begriffsbestimmung in einem beliebigen der Ansprüche 1 bis 6 in einer Impfstoffzusammensetzung, welche eine wässrige Phase und eine oder mehrere Öle, die aus den Mineralölen, den tierischen und den pflanzlichen Ölen gewählt sind, umfasst, wobei der Gewichtsanteil der Ölphase 10 % bis 90 % des Gesamtgewichts und vorzugsweise 30 bis 70 Gew.-% des Gesamtgewichts beträgt.

8. Verwendung gemäß der Begriffsbestimmung in einem beliebigen der Ansprüche 1 bis 7 in einer Impfstoffzusammensetzung, welche eine wässrige Phase umfasst und keine Ölphase umfasst.

9. Zusammensetzung, umfassend:
(i) mindestens ein Antigen oder mindestens ein Mittel, welches in vivo eine Verbindung erzeugt, die eine Aminosäuresequenz umfasst, und
(ii) mindestens eine Verbindung nach Formel (I):
R₁-O-(G)ₓ-H (I)
und
(iii) mindestens eine Verbindung nach Formel (II),
R₂-OH (II)
wobei R₂ unabhängig von R₁ für einen geradkettigen oder verzweigten, gesättigten oder ungesättigten Kohlenwasserstoffrest steht, der 8 bis 30 Kohlenstoffatome umfasst, wobei die Formel (I) der Begriffsbestimmung in einem beliebigen der Ansprüche 1 bis 6 entspricht.

10. Zusammensetzung gemäß der Begriffsbestimmung in Anspruch 9, welche darüber hinaus ein oder mehrere Öle umfasst, die aus den Mineralölen, den tierischen und den pflanzlichen Ölen gewählt sind, wobei der Gewichtsanteil der Ölphase 10 % bis 90 % des Gesamtgewichts und vorzugsweise 30 bis 70 Gew.-% des Gesamtgewichts beträgt.

11. Zusammensetzung gemäß der Begriffsbestimmung in Anspruch 10, wobei diese in Form einer Wasser-in-Öl-Emulsion (W/O), einer Ö1-in-Wasser-Emulsion (O/W), einer Wasser-in-Öl-in-Wasser-Emulsion (W/O/W) oder einer Mikroemulsion vorliegt.

12. Zusammensetzung gemäß der Begriffsbestimmung in Anspruch 9, welche keine Ölphase umfasst.

13. Zusammensetzung gemäß der Begriffsbestimmung in den Ansprüchen 9 bis 12, welche darüber hinaus ein oder mehrere organische Salze von wasserlöslichen Metallkationen umfasst, das/die aus Calciumgluconat, Mangangluconat, Manganglycerophosphat, Calciumglycerophosphat, Calciumfructophosphat, Manganfructophosphat, Aluminiumsalicylat und löslichem Aluminiumacetat gewählt ist/sind.

14. Zusammensetzung gemäß der Begriffsbestimmung in den Ansprüchen 9 bis 13, welche darüber hinaus eine sympathomimetische Verbindung umfasst, die aus Tyramin, Isoproterenol, L-Epinephrin, Norepinephrin, Ephedrin, Phenylephedrin und Salbutamol gewählt ist.

15. Zusammensetzung gemäß der Begriffsbestimmung in den Ansprüchen 9 bis 14, welche zur Durchführung einer therapeutischen Behandlung des menschlichen oder tierischen Körpers dient.

16. Zusammensetzung gemäß der Begriffsbestimmung in Anspruch 15, welche zur Durchführung einer therapeutischen Behandlung des menschlichen oder tierischen Körpers dient, und zwar auf parenteralem Wege durch subkutane, intramuskuläre oder intraperitoneale Injektion.

17. Zusammensetzung gemäß der Begriffsbestimmung in Anspruch 16, welche zur Durchführung einer therapeutischen Behandlung des menschlichen oder tierischen Körpers dient, und zwar über die Schleimhäute, insbesondere auf oralem, rektalem, nasalem oder vaginalem Wege.
